(19)

Europäisches
Patentamt
European
Patent Office
Office européen
des brevets

(11) **EP 4 442 282 A1**

(12) **EUROPEAN PATENT APPLICATION**

(43) Date of publication:
**09.10.2024 Bulletin 2024/41**

(21) Application number: **24168704.5**

(22) Date of filing: **05.04.2024**

(51) International Patent Classification (IPC):
*A61K 47/54* (2017.01)     *C07H 21/04* (2006.01)
*C12N 15/115* (2010.01)     *A61K 47/68* (2017.01)

(52) Cooperative Patent Classification (CPC):
**A61K 47/549; A61K 47/6889; C12N 15/115;**
C12N 2310/16; C12N 2310/336; C12N 2310/3513;
C12N 2310/3517

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **07.04.2023   KR 20230046232**

(71) Applicant: **POSTECH Research and Business
Development
Foundation
Pohang-si, Gyeongsangbuk-do 37673 (KR)**

(72) Inventors:
• **OH, Seung Soo**
  **37673 Gyeongsangbuk-do (KR)**
• **JO, Hyesung**
  **03168 Seoul (KR)**

(74) Representative: **Elkington and Fife LLP
Prospect House
8 Pembroke Road
Sevenoaks, Kent TN13 1XR (GB)**

Remarks:
The complete document including Reference
Table(s) and the Sequence Listing(s) can be
downloaded from the EPO website

(54)    **PREPARATION OF SITE-SPECIFIC POLYPEPTIDE CONJUGATES**

(57)    The present invention relates to a preparation of a polypeptide conjugate using an aptamer containing oxanine. In particular, the present invention relates to a composition for preparing a conjugate of a polypeptide, the composition comprising an aptamer containing oxanine, and a method of preparing a conjugate of a polypeptide using the same. The present invention also relates to a conjugate comprising a polypeptide and an aptamer containing oxanine, and a conjugate comprising a polypeptide and a drug.

EP 4 442 282 A1

**Description**

**CROSS-REFERENCE TO RELATED APPLICATIONS**

The present application is based on, and claims priority from, Korean Patent Applications No. 10-2023-0046232 filed on April 7 2023, the disclosure of which is hereby incorporated by reference herein in its entirety.

**TECHNICAL FIELD**

The present invention relates to a preparation of a polypeptide conjugate using an aptamer containing oxanine. In particular, the present invention relates to a composition for preparing a conjugate of a polypeptide, the composition comprising an aptamer containing oxanine, and a method of preparing a conjugate of a polypeptide using the same. The present invention also relates to a conjugate comprising a polypeptide and an aptamer containing oxanine, and a conjugate comprising a polypeptide and a drug.

This study was conducted with support from the Samsung Future Technology Incubation Program (Project No.: SRFC-MA2001-08).

**BACKGROUND OF THE INVENTION**

The emergence of diverse techniques in bioconjugate chemistry is rapidly changing the paradigm of biotechnology research and development. By chemically linking one molecule to another, we can create a completely new, bioactive molecule that successfully combines different functions of conjugate elements, which can be proteins, nucleic acids (NAs), lipids, carbohydrates or other biologically active molecules, and even whole cells. The functional integration is highly synergistic; for example, the potency of a cytotoxic drug embedded in antibody-drug conjugates (ADCs) is increased by 100-1000 fold more than when it acts alone. Indeed, technological advances driven by bioconjugate chemistry have been exceptionally influential across medical and pharmaceutical fields, creating high value-added industries. In the oncology market, six ADC drugs have been approved and marketed for the last 10 years.

Amidst the growing repertoire of bioconjugation techniques, there is an increasing need for chemical strategies that can effectively and site-specifically attach synthetic molecules to proteins. On a native protein surface, functional groups such as lysine and cysteine are present in large numbers, which not only hinders selectivity in conjugation but also leads to malfunctioning of the modified protein by undesirable conjugation to active sites. In addition, conventional bioconjugate methods require pretreatment processes such as genetic modification or chemical treatment, and as a result, they are indiscriminately expressed on the surface of the target rather than at a specific location, making selective labeling impossible. In this regard, for example, conventionally developed ADC products often exist heterogeneous mixtures including underconjugated antibodies, overconjugated antibodies, and even non-conjugated antibodies. Moreover, the location of drug attachment is not specific, which is known to affect the pharmacokinetics, safety, and efficacy profile of ADCs. Due to this uncertainty of ADC structure, there is a problem of failing to obtain drug approval and causing unpredictable side effects in patients.

Therefore, there is a need for the development of technology to produce bioactive conjugate molecules in which a desired substance selectively and efficiently binds to the exact desired site of the polypeptide.

**SUMMARY OF THE INVENTION**

The present invention relates to a preparation of a polypeptide conjugate using an aptamer containing oxanine.

An embodiment described herein provides a composition for preparing a conjugate of a polypeptide, the composition comprising an aptamer containing oxanine, wherein the aptamer specifically recognizes the polypeptide.

Another embodiment described herein provides a method of preparing a conjugate of a polypeptide, comprising mixing above composition with a polypeptide.

Another embodiment described herein provides a use of an aptamer containing oxanine for preparing a conjugate of a polypeptide, wherein the aptamer specifically recognizes the polypeptide.

Another embodiment described herein provides an aptamer containing oxanine for use in preparing a conjugate of a polypeptide, wherein the aptamer specifically recognizes the polypeptide.

Another embodiment described herein provides a method of preparing a conjugate of a polypeptide, comprising mixing an aptamer containing oxanine with a polypeptide.

Another embodiment described herein provides a conjugate comprising a polypeptide and an aptamer containing oxanine, wherein the aptamer specifically recognizes the polypeptide.

Another embodiment described herein provides a conjugate comprising a polypeptide and a drug, wherein the drug is conjugated to the polypeptide via an aptamer containing oxanine, and the aptamer specifically recognizes the polypeptide.

## BRIEF DESCRIPTION OF THE DRAWINGS

Fig. 1 is a diagram schematically illustrating the preparation of a polypeptide conjugate using an aptamer containing oxanine, according to an embodiment of the present disclosure.

Fig. 2 shows SDS-PAGE and electrophoresis results confirming the preparation of a thrombin conjugate using an aptamer containing oxanine.

Fig. 3 shows the conversion rate (%) of aptamer HD1 and aptamer HD22 over time in the production of a thrombin conjugate using an aptamer containing oxanine.

Fig. 4 shows SDS-PAGE and electrophoresis results confirming the preparation of a conjugate of PTK7 using an aptamer containing oxanine.

Fig. 5 shows SDS-PAGE and electrophoresis results confirming the preparation of a conjugate of nucleolin using an aptamer containing oxanine.

Fig. 6 shows SDS-PAGE and electrophoresis results confirming the preparation of a conjugate of Fc dimer using an aptamer containing oxanine.

Fig. 7 shows SDS-PAGE and electrophoresis results confirming the preparation of a conjugate of an antibody using an aptamer containing oxanine.

Fig. 8 shows SDS-PAGE and electrophoresis results confirming the preparation of an antibody-drug conjugate using an aptamer containing oxanine, according to an embodiment of the present disclosure.

Fig. 9 shows the results of confirming the antigen (HER) binding activity of an antibody-drug conjugate using an aptamer containing oxanine, according to an embodiment of the present disclosure.

Fig. 10 shows the results of confirming the FcγR binding (FcγRIIIa binding) activity of an antibody-drug conjugate using an aptamer containing oxanine, according to an embodiment of the present disclosure.

Fig. 11 shows the results of confirming the pH-dependent FcRn binding activity of an antibody-drug conjugate using an aptamer containing oxanine, according to an embodiment of the present disclosure.

Fig. 12 shows the results of confirming the target cell killing effect of an antibody-drug conjugate using an aptamer containing oxanine, according to an embodiment of the present disclosure (drug concentration 20 nM).

Fig. 13 shows the results of confirming the target cell killing effect of an antibody-drug conjugate using an aptamer containing oxanine, according to an embodiment of the present disclosure (drug concentration 10 nM).

Fig. 14 shows the results of confirming the target cell killing effect of an antibody-drug conjugate using an aptamer containing oxanine, according to an embodiment of the present disclosure (drug concentration 5 nM).

## DETAILED DESCRIPTION OF THE INVENTION

### Overview

The present inventors provide a technology that can form a conjugate with high affinity and selectivity at a desired site of a target polypeptide without modifying the target polypeptide using the properties of aptamers that specifically recognize polypeptides and the properties of oxanine that can react directly with amine groups without using a chemical activation process or reactive linker. In particular, when oxanine is introduced into the sequence of an aptamer that specifically recognizes a target polypeptide, the aptamer acts as a carrier for oxanine, allowing oxanine to be accurately located at a specific site of the polypeptide recognized by the aptamer. Then, at that specific site, oxanine can react with the amine group of the side chain of the amino acid (e.g., lysine) of the target polypeptide to form an amide bond.

Accordingly, by attaching a second moiety, such as a drug, a detectable label, a small molecule, a polymer, etc., to an aptamer containing oxanine, the second moiety may be indirectly conjugated to the polypeptide through the aptamer containing oxanine. Thus, the present disclosure can be used to prepare a variety of polypeptide conjugates. In an embodiment, when a drug is attached to an aptamer containing oxanine and treated with an antibody (polypeptide) that is specifically recognized by the aptamer, oxanine forms an amide bond at a specific site on the antibody that is specifically recognized by the aptamer. As a result, a drug-aptamer-antibody conjugate (ADC) is formed and can be used for therapeutic purposes. In another embodiment, when a fluorophore (detectable label) is attached to an aptamer containing oxanine and treated with an antibody (polypeptide) that is specifically recognized by the aptamer, oxanine forms an amide bond at a specific site on the antibody that is specifically recognized by the aptamer. As a result, a fluorophore-aptamer-antibody conjugate is formed and can be used for diagnostic or detection purposes.

In this regard, aptamers containing oxanine herein may serve as a reactive agent or a crosslinker that allows a second moiety such as a drug, a detectable label, a small molecule, a polymer, etc. to bind to a desired position of the polypeptide in preparing various polypeptide conjugates, such as, but not limited to, antibody-drug conjugates (ADCs),

This site-specific conjugate manufacturing strategy is based on the modular structure which simultaneously performs 1) lock-and-key binding and 2) proximity-enhanced reaction:

1) Lock-and-key binding by aptamer

Aptamers are nucleic acids such as DNA and RNA, and are known to fold into a variety of 3D structures, which offers them the ability to bind target molecules with high affinity and specificity. Aptamers can be developed through in vitro selection, typically referred to as in vitro evolution, or SELEX, or modified methods thereof. Once the aptamer capable of recognizing a specific target is generated, it can serve as a reactive moiety carrier to the reaction site of the target.

2) Proximity-enhanced reaction by oxanine

Several modified nucleobases are known that their activities dramatically increase as they approach the targeted functional groups that are plentiful in nature (e.g. amine, thiol, hydroxyl, etc.). The present inventors introduced oxanine (reactive moiety), one of the modified nucleobases, into the aptamer in a sequence-specific manner. Thus, an aptamer containing oxanine can bind to a specific point of an intact target with high affinity and selectivity. Specifically, by recognizing the substrate of the aptamer, oxanine (reactive moiety) introduced into the aptamer can be accurately located near the target site. Therefore, even if several functional groups exist on the target, coupling occurs only at the correct location. Accordingly, site-selective conjugation can be induced by appropriately selecting an aptamer that specifically recognizes a region including the position to be conjugated in the target polypeptide.

Accordingly, the present invention can provide a novel platform that:

- forms stable chemical bonding only at the desired site with ultra-high specificity and efficiency;
- targets natural polypeptides, including native antibodies, without any chemical or genetic modification;
- produces homogeneous conjugated products, maximizing therapeutic efficacy, safety, and stability;
- has a simple and convenient process, as there is no need to apply prior modifications such as genetic or chemical modification to the polypeptide; and
- can be a universal strategy that is generally applicable to a variety of different polypeptides.

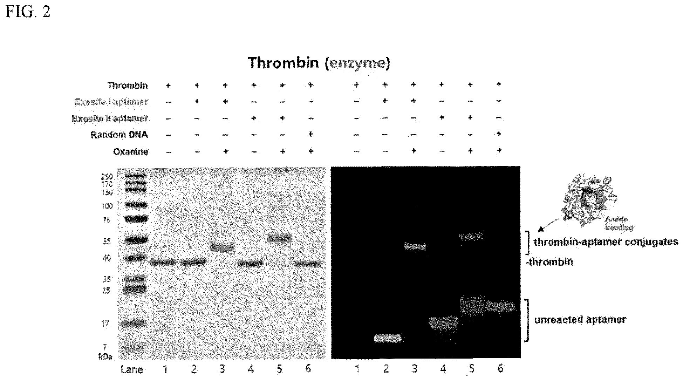

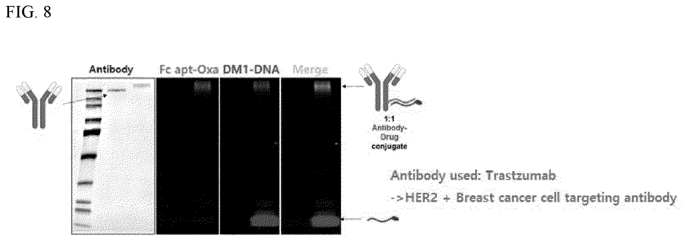

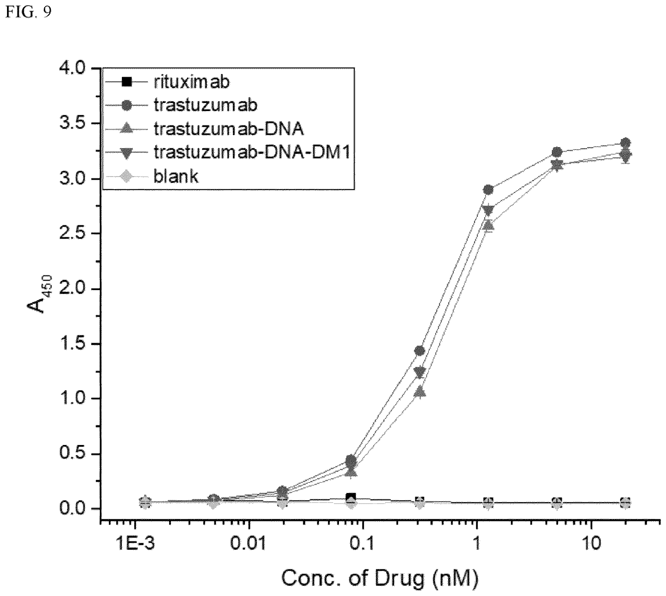

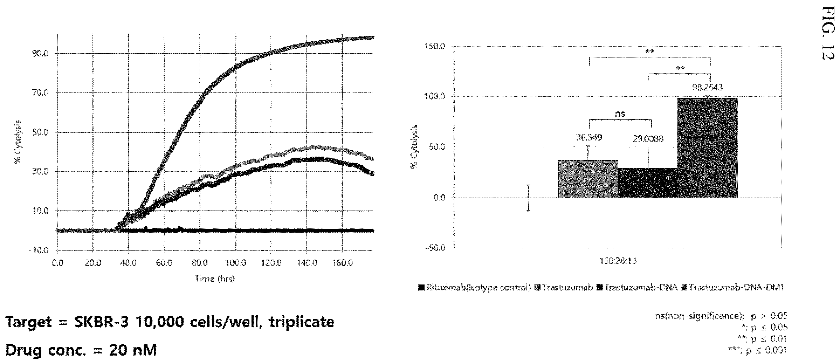

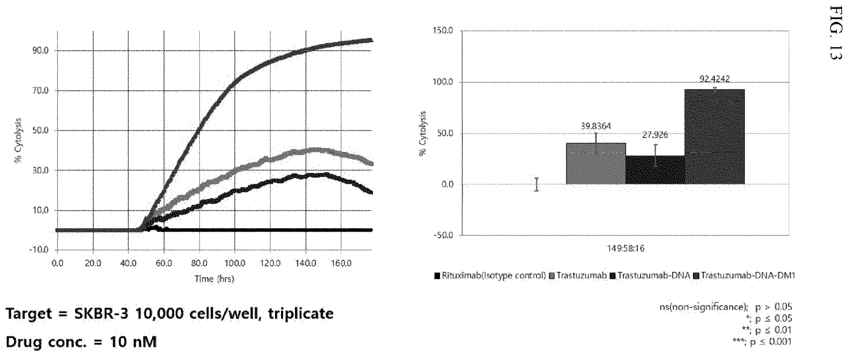

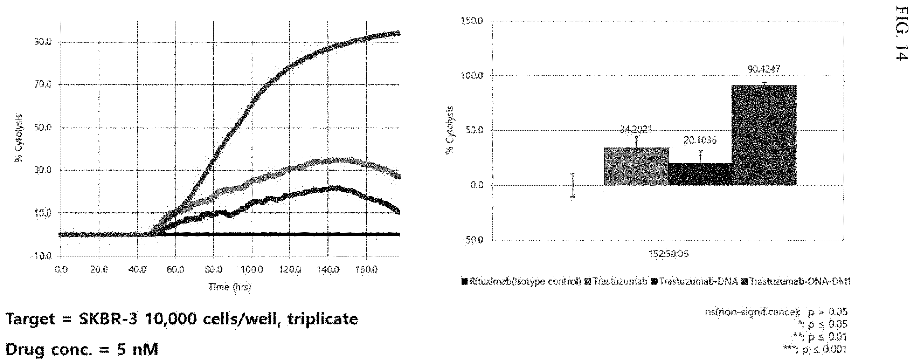

In the examples of the present disclosure, it was confirmed that covalent bonding was induced with high ultra-high efficiency by a simple one-pot process of introducing oxanine into an aptamer that specifically recognizes various polypeptides, such as enzymes (e.g., thrombin), membrane receptor proteins (e.g., PTK7), tumor-related nucleolar proteins (e.g., nucleolin), Fc dimer proteins, and full-length antibodies (e.g., trastuzumab), etc., and then mixing it with the polypeptide (See Figs. 2 to 7). Furthermore, an antibody-aptamer-drug conjugate was prepared by attaching a drug to an aptamer in the antibody-aptamer conjugate (Fig. 8), and the conjugate exhibited drug-induced cytotoxicity (Figs. 9 to 11) while maintaining the original representative functions of the antibody, such as antigen-binding ability, FcγR-binding ability, and FcRn-binding ability (Figs. 12 to 14).

Therefore, the present inventors confirmed that using an aptamer containing oxanine can be a simple, powerful, and general approach to produce a conjugate site-selectively at a specific site of the target through target recognition by the lock-and-key model using an aptamer and chemical reaction with the target substance using oxanine, and thus completed the present invention.

According to one aspect of the present invention, there is provided a composition for preparing a conjugate of a polypeptide, the composition comprising an aptamer containing oxanine, wherein the aptamer specifically recognizes the polypeptide.

According to another aspect of the present invention, there is provided a method of preparing a conjugate of a polypeptide, comprising mixing above composition with a polypeptide.

According to another aspect of the present invention, there is provided a conjugate comprising a polypeptide and an aptamer containing oxanine, wherein the aptamer specifically recognizes the polypeptide.

According to another aspect of the present invention, there is provided a conjugate comprising a polypeptide and a drug, wherein the drug is conjugated to the polypeptide via an aptamer containing oxanine, and the aptamer specifically recognizes the polypeptide.

Hereinafter, the present invention will be described in more detail.

Where the terms "comprise", "comprises", "comprised" or "comprising" are used in this specification (including the claims) they are to be interpreted as specifying the presence of the stated features, integers, steps or components, but not precluding the presence of one or more other feature, integer, step, component or group thereof.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention before the priority date of each claim of this application.

## Aptamer

The term "aptamer" as used herein refers to a single-stranded nucleic acid that has a stable tertiary structure and the ability to bind to a target molecule with high affinity and specificity. In the present disclosure, the aptamer may be DNA, RNA, modified nucleic acid, or a mixture thereof.

As mentioned above, as the aptamer herein specifically recognizes the target, it can serve to transport oxanine so that oxanin can be accurately located at a specific site of the polypeptide recognized by the aptamer. Therefore, the aptamer of the present disclosure is characterized by containing oxanine, one of the nucleobases, in its sequence. Oxanine may be introduced at any position in the sequence of the target recognition site of the aptamer.

The aptamer herein may be 10 to 100, or 10 to 90, or 10 to 80, or 10 to 70, or 10 to 60, or 10 to 50, or 20 to 100, or 20 to 90, or 20 to 80, or 20 to 70, or 20 to 60, or 20 to 50, or 30 to 100, or 30 to 90, or 30 to 80, or 30 to 70, or 30 to 60, or 30 to 50, or 40 to 100, or 40 to 90, or 40 to 80, or 40 to 70, or 40 to 60, or 40 to 50 nucleotides in length, but is not limited thereto.

Each of the nucleotides contained in the aptamer may be the same or different, and may be a nucleotide containing a hydroxyl group at the 2' position of ribose (e.g., ribose of pyrimidine nucleotide) (i.e., an unsubstituted nucleotide) or a nucleotide in which the hydroxyl group at the 2' portion of the ribose is substituted with any atom or group. Examples of such nucleotide substituted with any atom or group include a nucleotide substituted with a hydrogen atom, a fluorine atom, an -O-alkyl group (e.g., -O-Me group), an -O-acyl group (e.g., -OCHO group), an amino group (e.g., $-NH_2$ group), etc. Further, in the aptamer, at least one (e.g., 1, 2, 3, or 4) nucleotide may contain, at the 2' portion of the ribose, a hydroxyl group or at least two (e.g., 2, 3, or 4) atoms or groups selected from any of the foregoing atoms or groups, for example, a hydrogen atom, a fluorine atom, a hydroxyl group and an -O-Me group. Further, in the aptamer, all of the nucleotides may contain, at the 2' portion of the ribose, a hydroxyl group or the same group selected from any of the foregoing atoms or groups, for example, a hydrogen atom, a fluorine atom, a hydroxyl group and an -O-Me group.

In one embodiment, in the aptamer, a sugar residue (e.g., ribose or deoxyribose) of each nucleotide may be modified to increase binding to the target or stability. As a site to be modified in the sugar residue, for example, the oxygen atom of the 2', 3', and/or 4' positions of the sugar residue may be replaced with another atom. Types of the modification may include, for example, fluorination, O-alkylation (e.g., O-methylation, O-ethylation), O-arylation, and S-alkylation (e.g., S-methylation, S-ethylation), S-arylation, amination (e.g., $-NH_2$). Such alterations in the sugar residue can be performed by a method known per se (See, for example, Sproat et al., Nucle. Acid. Res. 19, 733-738, 1991; Cotton et al., Nucl. Acid. Res. 19, 2629-2635, 1991; Hobbs et al., Biochemistry 12, 5138-5145, 1973).

In another embodiment, in the aptamer, a nucleic acid base (e.g., purine, pyrimidine) may be modified (e.g., chemical substitution) to increase binding to the target. Such modifications include, for example, 5-position pyrimidine modifications, 6- and/or 8-position purine modifications, modifications with exocyclic amines, substitution with 4-thiouridine, and substitution with 5-bromo or 5-iodo-uracil. In addition, the phosphate group contained in the aptamer may be modified to confer resistance to nuclease and hydrolysis. For example, the P(O)O group may be substituted with P(O)S (thioate), P(S)S (dithioate), $P(O)NR_2$ (amidate), P(O)R, R(O)OR', CO or $CH_2$ (formacetal) or 3'-amine (-NH-$CH_2$-$CH_2$-) [wherein each R or R' is independently H or a substituted or unsubstituted alkyl (e.g., methyl, ethyl)]. Examples of a linking group include -O-, -N-, or -S-, and nucleotides can bind to an adjacent nucleotide via these linking groups. Modifications may also include 3' and 5' modifications, such as capping. The alterations may also include alterations such as capping at 3' and 5'. Further, modifications may be performed by adding to an end a polyethyleneglycol, amino acid, peptide, inverted dT, nucleic acid, nucleosides, Myristoyl, Lithocolic-oleyl, Docosanyl, Lauroyl, Stearoyl, Palmitoyl, Oleoyl, Linoleoyl, other lipids, steroids, cholesterol, caffeine, vitamins, pigments, fluorescent substances, anticancer agent, toxin, enzymes, radioactive substance, biotin and the like. For such modifications, see, for example, U.S. Pat. Nos. 5,660,985 and 5,756,703.

The aptamer herein may be an aptamer known in the art to specifically recognize a specific target, or may be manufactured and selected to specifically recognize a specific target.

Typically, aptamers may be selected through an in vitro evolution method called Systematic evolution of ligands by exponential enrichment (SELEX), but are not limited thereto. In one embodiment, in the SELEX process, a nucleic acid library of various forms ($>10^{15}$) is first created using DNA synthesis and in vitro transcription method (in the case of RNA), and from this, only nucleic acid constructs that can bind to the desired target molecule are selected. For example, through methods such as affinity chromatography, non-binding nucleic acid constructs are discarded (washed) and only those that bind to the target molecule can be selectively obtained. Finally, the nucleic acid constructs are separated from the target molecule (elution) and are amplified. Using the nucleic acid constructs obtained in this way, the above processes may be repeated 5 to 15 times to discover an aptamer that shows excellent binding and specificity.

In another embodiment, a post-SELEX process may be performed to improve the aptamers obtained through SELEX into more stable and strong aptamers. A typical example is to replace the ribose 2'-OH of RNA aptamer with a 2'-F, 2'-NH$_2$, or 2'-O-methyl group. Through this modification, it is possible to obtain an aptamer having an excellent resistance to a nucleic acid degrading enzyme and an increase in stability in blood of more than 10,000 times. In addition, the aptamer herein may be obtained by various aptamer production methods known in the art (See, for example, U.S. Patent Nos. 5,270,163; 5,475,096; 5,705,337; 5,660,985; 5,580,737; 6,376,190; 7,947,447; 7,855,054; and 8,409,795; U.S. Patent Publication Nos. 20070166740; and 20090098549, etc.). Alternatively, aptamers may be obtained more efficiently using the in vitro evolution method previously reported by the inventor of the present application (See Angew Chem Int Ed Engl. 2016 December 05; 55(49): 15258-15262).

**Aptamer containing oxanine**

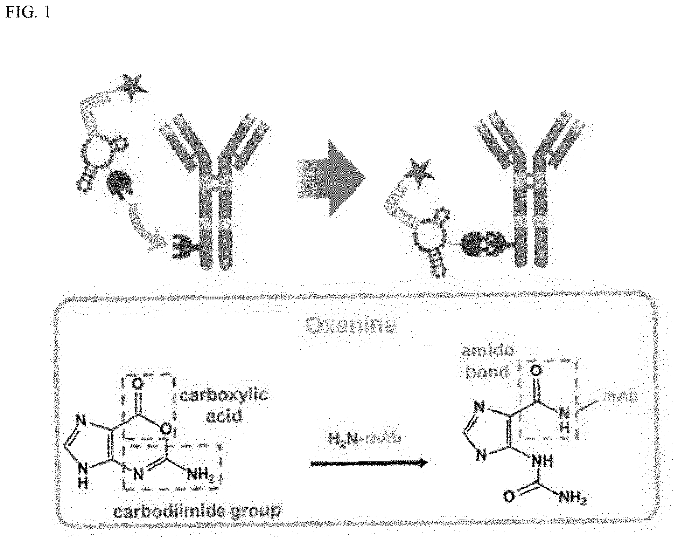

Oxanine is a ring-shaped nucleobase produced by nitrosation of guanine. As shown in Fig. 1, as there is a carboxyl group bound to carbodiimide in the oxanine ring, oxanine can react directly with an amine group without using a chemical activation process or a reactive linker. Accordingly, oxanine can be covalently attached to a polypeptide by reacting with an amine group of an amino acid side chain, for example, an amine group of a lysine side chain to form an amide bond.

The form in which oxanine is combined with ribose (furanose type) is referred to as oxanosine, the form in which oxanine is combined with deoxyribose (furanose type) is referred to as deoxyoxanosine. Oxanine can be obtained, for example, by reacting deoxyguanosine with NO and/or NaNO2 under acidic conditions to obtain deoxyoxanosine form, and then acid hydrolyzing the deoxyguanosine, but is not limited thereto.

Oxanine may be used in any form as long as the carboxyl group activated by carbodiimide maintains reactivity with an amine group. For example, oxanine may be used in a combined form with a 5-carbon sugar such as ribose or deoxyribose (i.e., oxanosine or deoxyoxanosine), but is not limited thereto.

Oxanine can be introduced at any position in the sequence of the target recognition site of the aptamer. "Introduction" or "integration" of oxanine into an aptamer means that oxanine is inserted at any position in the nucleic acid sequence of the aptamer. For example, oxanine may be inserted in the middle or at the end of the nucleic acid sequence of aptamer, and one or more, for example, 2, 3, 4, 5, 6, 7, 8, 9, or 10 or more oxanines may be inserted at any position as needed, but are not limited thereto. In one embodiment, in the examples herein, an enzymatic strategy was established to introduce oxanine into the aptamer using dOTP (2'-deoxyoxanosine triphosphate) and DNA polymerase. As oxanine is a guanosine analogue that forms hydrogen bonds with cytosine, dOTP can be incorporated into the aptamer strand via DNA chain extension using a cytosine-containing DNA template, DNA polymerase, and primers from the 5' end to the desired position. The first extension step is performed in a reaction system containing only dOTP, followed by normal extension steps in the presence of the required dNTP (N is A, T, C or G). After the polymerase reaction, only a single-stranded aptamer containing oxanine can be obtained by removing the template DNA. The present inventors confirmed that dOTPs can be inserted into DNA, RNA, or even modified DNA/RNA hybrid strand, indicating the availability of various aptamers for this approach. Moreover, the position and number of dOTPs to be inserted can be flexibly designated by designing template sequence. Through this strategy, oxanine can be introduced at any position in the nucleic acid sequence of the aptamer, but the present invention is not limited thereto.

**Conjugate**

The term "conjugate" refers to a substance in which a first moiety is stably associated or combined with a second moiety. For the purposes herein, the first moiety and the second moiety may be conjugated via an aptamer containing oxanine (reactive moiety). That is, the first moiety and the second moiety may each be bound (e.g., covalently linked) to an aptamer containing oxanine, and thus the first moiety and the second moiety can be indirectly conjugated through an aptamer containing oxanine.

Specifically, the first moiety may be a polypeptide specifically recognized by the aptamer. When oxanine is introduced into the sequence of an aptamer that specifically recognizes a target polypeptide, the aptamer acts as a carrier for oxanine, allowing oxanine to be accurately located at a specific site of the polypeptide recognized by the aptamer. Then, at that specific site, oxanine can react with the amine group of the side chain of the amino acid (e.g., lysine) of the target polypeptide to form an amide bond. Therefore, an aptamer containing oxanine can be bound to the polypeptide, which is the first moiety, by a covalent bond between oxanine and the polypeptide.

Meanwhile, the second moiety can bind to an aptamer containing oxanine. The second moiety may be, but is not limited to, a drug to be conjugated to the polypeptide (the first moiety), a detectable label, a small molecule, a polymer, etc. The second moiety may be bound (e.g., covalently linked) to any portion of the aptamer containing oxanine. For example, it may bind to a site other than the target recognition site in the structure of the aptamer (e.g., a site that supports the three-dimensional structure of the aptamer without being directly involved in target recognition). A person skilled in the art can appropriately perform the binding of the second moiety to the aptamer based on known knowledge in the art. For example, the binding between the aptamer and the second moiety may be covalent or non-covalent, but is not limited thereto. Further, the binding between the aptamer and the second moiety may be a direct binding or an indirect binding through a linker, but is not limited thereto. Further, the binding between the aptamer and the second moiety may be indirect binding through an oligonucleotide complementary to the aptamer, but is not limited thereto.

In one embodiment, the aptamer and the second part may be easily and directly linked through enzymatic synthesis or chemical synthesis using one or more functional groups present on each of the aptamer and the second part. Alternatively, direct binding can be induced by introducing a functional group into the aptamer and/or the second moiety for direct binding. In another embodiment, if a drug has the property of intercalating into the double-stranded DNA structure of nucleic acid, the aptamer can be bound to the drug using this property. These drugs may be hydrophobic compounds having a phenyl group, for example, taxanes, camptotheca extract, anthracyclines, specifically paclitaxel, docetaxel, camptothecin, doxorubicin, daunorubicin, epirubicin, idarubicin, etc., but are not limited thereto.

In another embodiment, the aptamer and the second moiety may be linked indirectly through a linker. For example, the linker may be a cleavable linker or a non-cleavable linker. Cleavable linkers may be separated in vivo by the biological environment. The cleavage may come from any process without limitation, e.g., enzymatic, reductive, pH, etc. The linker may be relatively stable in the neutral pH environment of blood, but may be cleaved after the conjugate enters the lower pH environment inside the cell. In another embodiment, the linker may be cleaved by the action of in vivo enzymes, for example proteases. Enzymatically cleaveable groups may include nucleotide sequences that end with a natural nucleotide and are attached at their carboxyl terminus to the linker. Further, the linker may be a peptidic or non-peptidic linker. The linker may be a molecule containing a spacer moiety and two or more functional group moieties, where one functional group is for binding to the aptamer and the other functional group is for binding to the second moiety. The functional group is a group that connects the aptamer and the second moiety and may include all functional groups that can bind the aptamer and the second moiety. For example, the functional group may be an amine group ($-NH_2$), a sulfhydryl group (-SH), -CH, -COOH, alkenyl, phosphate, sulfate, heterocyclic NH, alkyne, and ketone, but is not limited thereto.

In another embodiment, the second moiety may bind to the oligonucleotide complementary to the aptamer, and the aptamer may hybridize with the oligonucleotide complementary thereto, resulting in the second moiety being able to bind to the aptamer (See Example 6).

Accordingly, in a preferred embodiment, a conjugate herein is a polypeptide conjugate wherein the first moiety is a polypeptide and the polypeptide is conjugated to the second moiety via an aptamer containing oxanine. The second moiety conjugated to the polypeptide may include, but is not limited to, a drug, a detectable label, a small molecule, a polymer, etc. The second moiety is bound to an aptamer containing oxanine, and at a specific site of the polypeptide specifically recognized by the aptamer, oxanine forms a covalent bond with an amino acid residue (e.g., lysine) containing an amine group in the side chain. As a result, the second moiety can be conjugated to a specific desired site of the polypeptide.

In addition, the polypeptide may be conjugated to one or more second moieties, e.g., 2, 3, 4, 5, 6, 7, 8, 9 or 10 or more second moieties. In one example, an aptamer to which one or more second moieties, such as 2, 3, 4, 5, 6, 7, 8, 9 or 10 or more second moieties are attached may be covalently linked to a polypeptide. In another example, one or more aptamers, such as 2, 3, 4, 5, 6, 7, 8, 9 or 10 or more aptamers to which a second moiety is attached may be covalently linked to a polypeptide. In another example, one or more aptamers to which one or more second moieties are attached may be covalently linked to the polypeptide.

## Polypeptide

The term "polypeptide" is used interchangeably with "peptide" and "protein" and refers to a polymeric form of amino acids of any length. "Polypeptide," "peptide," and "protein" include both naturally-occurring polypeptides or fragments thereof and synthetic polypeptides or fragments thereof. A polypeptide herein that form a conjugate may include, but are not limited to, a therapeutic polypeptide, an antibody or a fragment thereof, a therapeutic antibody, a diagnostic antibody, etc. Representative examples are described below.

### Therapeutic polypeptide

A therapeutic polypeptide can be conjugated for a variety of purposes, including, for example, increasing serum half-life, increasing biological activity or bioavailability, increasing or decreasing immunogenicity, and reducing side effects, etc.

A therapeutic polypeptide includes, but is not limited to, an enzyme, a cytokine, a chemokine, a growth factor, a hormone, a receptor, a tumor-related protein, etc. In particular, it includes, but is not limited to, erythropoietin (EPO), growth hormone, follicle-stimulating hormone (FSH), interferon (e.g., IFN-$\gamma$, IFN-$\alpha$, IFN-$\beta$, IFN-$\omega$, IFN-$\tau$, consensus interferon, etc.), insulin, insulin-like growth factor (e.g., IGF-I, IGF-II, etc.), blood factors (e.g., factor X, tissue plasminogen activator (TPA), factor VIIa, factor VIII, factor IX, $\beta$-globin, hemoglobin, etc.), colony stimulating factor (e.g., granulocyte-CSF(G-CSF), macrophage- CSF(M-CSF), granulocyte- macrophage-CSF (GM-CSF), granulocyte-monocyte colony stimulating factor, megakaryocyte monocyte colony-stimulating factor, etc.), transforming growth factor (e.g., TGF-beta, TGF-alpha), interleukin (e.g., IL-1, IL-2, IL-3, IL-4, IL-5, IL-6, IL-7, IL-8, IL-9, IL-12, etc.), platelet-derived growth factor (PDGF), fibroblast growth factor (FGF, e.g., aFGF, bFGF), glial cell line-derived neurotrophic factor (GDNF), nerve growth factor (NGF), stem cell factor, keratinocyte growth factor, hepatocyte growth factor, etc.), soluble receptor (e.g., TNF-$\alpha$--binding soluble receptor, soluble VEGF receptor, soluble interleukin receptor, soluble $\gamma/\delta$ T cell receptor, etc.), enzyme (e.g., thrombin, $\alpha$-glucosidase, $\beta$-glucocerebrosidase, etc.), chemokine (e.g., IP-10, Mig, Gro$\alpha$/IL-8, RANTES, MIP-1$\alpha$, MIP-1$\beta$, MCP-1, PF-4, etc.), angiogenic agent (e.g., vascular endothelial growth factor (VEGF)), antiangiogenic agent (e.g., solubl VEGF receptor), protein vaccine, neurotropic peptide, thrombolytic agent, atrial natriuretic peptide, bone morphogenetic protein, thrombopoietin, relaxin,, glial fibrillary acidic protein, tumor necrosis factor, neutrophil tropism factor, angiogenin, angiotropin, fibrin, hirudin.

## *Antibody or a fragment thereof*

The term "antibody" as used herein collectively refers to a protein that specifically binds to a specific antigen or epitope, and is used in the broadest sense, and it may be a protein produced in the immune system by stimulation of an antigen or a protein synthesized chemically or prepared recombinantly. The type thereof is not particularly limited. Specifically, the antibody encompasses monoclonal antibodies (including full-length monoclonal antibodies), polyclonal antibodies, multispecific antibodies (e.g., bispecific antibodies), synthetic antibodies (also called antibody mimetics), chimeric antibodies, humanized antibodies, human antibodies, or antibody fusion proteins (also called antibody conjugates), provided such antibodies exhibit a desired biological activity. Furthermore, the antibody may be a therapeutic antibody or a diagnostic antibody, but is not limited thereto.

The term "monoclonal antibody" refers to an antibody molecule obtained from a population of substantially homogeneous antibodies. The monoclonal antibody displays a single-binding specificity and affinity for a specific epitope. The term "polyclonal antibody" refers to an antibody mixture comprising two or more monoclonal antibodies that bind to different epitopes of the same antigen. The polyclonal antibody is capable of reacting with a plurality of epitopes. The term "chimeric antibody" refers to an antibody obtained by recombining the variable region of a non-human antibody and the constant region of a human antibody. The chimeric antibody provides greatly improved immune response compared to non-human antibody. The term "humanized antibody" refers to an antibody formed by grafting all or some of a CDR sequence of a non-human antibody into a human antibody. For example, CDRs of a murine monoclonal antibody may be recombined with human antibody-derived framework region (FR) to prepare a humanized variable region, and then the humanized variable region may be recombined with a constant region of a desired human antibody to prepare a humanized antibody, but the present invention is not limited thereto. The term "human antibody" refers to an antibody that is completely free of parts derived from non-human animals. In general, a human antibody refers to an antibody in which both constant region and variable region including CDR and FR regions are derived from human germline immunoglobulin sequences.

An intact antibody (e.g., IgG type) has a structure with two full-length light chains and two full-length heavy chains in a Y shape, and is a homodimer of heterodimers where each heterodimer is composed of one copy of the heavy chain and one copy of the light chain. Each light chain is linked to a corresponding heavy chain via a disulfide bond. The constant region of an antibody is divided into a heavy-chain constant region and a light-chain constant region. The heavy-chain constant region is of a gamma ($\gamma$), mu ($\mu$), alpha ($\alpha$), delta ($\delta$), or epsilon ($\epsilon$) type, and has gamma1 ($\gamma$1), gamma2 ($\gamma$2), gamma3 ($\gamma$3), gamma4 ($\gamma$4), alpha1 ($\alpha$1) or alpha2 ($\alpha$2) as its subclass. The light chain constant region is of either a kappa ($\kappa$) or lambda ($\lambda$) type.

As used herein, the term "heavy chain" may be intended to encompass a full-length heavy chains and fragments thereof, wherein the full-length heavy chain may comprise a variable region $V_H$ including amino acid sequences sufficient to provide specificity to antigens, three constant regions $C_{H1}$, $C_{H2}$, and $C_{H3}$, and a hinge.

The term "light chain" may be intended to encompass full-length light chains and fragments thereof, wherein the full-length light chain may comprise a variable region $V_L$ including amino acid sequences sufficient to provide specificity to antigens, and a constant region $C_L$.

The term "complementarity-determining region (CDR)" refers to a region in a variable region of an antibody, which imparts binding specificity or binding affinity to an antigen. Generally, there are three CDRs (CDR-H1, CDR-H2, CDR-H3) in a heavy chain variable region, and three CDRs (CDR-L1, CDR-L2, CDR-L3) in a light chain variable region. The CDRs may provide key contact residues for an antibody or a fragment thereof binding to an antigen or an epitope. The term "framework region (FR)" refers to non-CDR regions of variable regions of heavy and light chains. Generally, there are four FRs (FR-H1, FR-H2, FR-H3, and FR-H4) in a heavy chain variable region, and four FRs (FR-L1, FR-L2, FR-L3 and FR-L4) in a light chain variable region. The precise amino acid sequence boundaries of a given CDR or FR may be readily determined using any of a number of well-known schemes, such as Kabat numbering scheme, Chothia numbering scheme, Contact numbering scheme, IMGT numbering scheme, Aho numbering scheme, AbM numbering scheme, etc.

The term "variable region" refers to a domain of a heavy chain or light chain of an antibody, which is involved in binding of the antibody to an antigen. The heavy chain variable (VH) region and the light chain variable (VL) region generally have the similar structure, and each domain includes four conserved framework regions (FRs) and three CDRs.

The term "fragment of an antibody" refers to a any fragment of an antibody that lacks at least some of the amino acids present in the full-length chain of the antibody. Examples of the fragment of an antibody may include Fc, F(ab')$_2$, single chain variable fragments (scFvs) (e.g., scFv, (scFv)$_2$, etc.), fragment antigen binding (Fab) (e.g., Fab, Fab', F(ab')$_2$, etc.), domain antibodies, peptibodies, minibodies, intrabodies, diabodies, triabodies, tetrabodies, and single-chain antibodies, etc., but are not limited thereto. Further, the fragment of an antibody may be scFv, or a fusion polypeptide (scFv-Fc), in which scFv is fused with an Fc region of an immunoglobulin (e.g., IgA, IgD, IgE, IgG (IgG1, IgG2, IgG3, IgG4), IgM, etc.), or a fusion polypeptide (scFv-Ck (kappa constant region) or scFv-Cλ (lambda constant region), in which scFv is fused with a light chain constant region (e.g., kappa or lambda), but is not limited thereto.

*Therapeutic antibody*

The term "therapeutic antibody" refers to an antibody that is used in the treatment of disease. A therapeutic antibody may have various mechanisms of action. A therapeutic antibody may bind and neutralize the normal function of a target. For example, a monoclonal antibody that blocks the activity of the protein needed for the survival of a cancer cell causes the cell's death. Another therapeutic monoclonal antibody may bind and activate the normal function of a target. For example, a monoclonal antibody can bind to a protein on a cell and trigger an apoptosis signal. Finally, if a monoclonal antibody binds to a target expressed only on diseased tissue, conjugation of a toxic payload (effective agent), such as a chemotherapeutic or radioactive agent, to the monoclonal antibody can create an agent for specific delivery of the toxic payload to the diseased tissue, reducing harm to healthy tissue.

The therapeutic antibody may be an antibody used for the treatment of various diseases, such as cancer (e.g., hematological cancer and solid cancer), immune disease, neurological disease, vascular disease, or infectious disease. Antigens for these antibodies may include tumor antigens such as tumor-associated antigen (TAA), tumor-specific antigen (TSA) or tumor-derived neoantigen; antigens of infectious agents, such as antigens derived from viruses, bacteria, parasites, or fungi; autoantigens known or suspected to induce autoimmunity; or a peptide derived from an allergen known or suspected to cause allergy, but is not limited thereto.

For example, such an antibody may be an antibody against ALK, adhesion related kinase receptor (e.g., Axl), ERBB receptors (e.g., EGFR, ERBB2, ERBB3, ERBB4), erythropoietin-producing hepatocellular (EPH) receptors (e.g., EphA1, EphA2, EphA3, EphA4, EphA5, EphA6,EphA7, EphA8, EphB 1, EphB2, EphB3, EphB4, EphB5, EphB6), fibroblast growth factor (FGF) receptors (e.g., FGFR1, FGFR2, FGFR3, FGFR4, FGFR5), Fgr, IGF1R, insulin receptors, LTK, M-CSFR, MUSK, platelet-derived growth factor (PDGF) receptors (e.g., PDGFR-A, PDGFR-B), RET, ROR1, ROR2, ROS, RYK, vascular endothelial growth factor (VEGF) receptors (e.g., VEGFR1/FLT1, VEGFR2/FLK1, VEGF3), tyrosine kinase with immunoglobulin-like and EGF-like domains (TIE) receptors (e.g., TIE-1, TIE-2/TEK), Tec, TYRO10, insulin-like growth factor (IGF) receptors (e.g., INS-R, IGF-IR, IR-R), Discoidin Domain (DD) receptors (e.g., DDR1, DDR2), receptor for c-Met (MET), recepteur d'origine nantais (RON, also known as macrophage stimulating 1 receptor), Flt3 (fins-related tyrosine kinase 3), colony stimulating factor 1 (CSF 1) receptor, receptor for c-kit (KIT or SCFR), insulin receptor related (IRR) receptors, CD19, CD20, HLA-DR, CD33, CD52, G250, GD3, PSMA, CD56, CEA, Lewis Y antigen, or IL-6 receptor, but is not limited thereto.

*Diagnostic antibody*

The term "diagnostic antibody" refers to an antibody that is used as a diagnostic reagent for a disease. The diagnostic antibody may bind to a target that is specifically associated with, or shows increased expression in, a particular disease. The diagnostic antibody may be used, for example, to detect a target in a biological sample from a patient, or in diagnostic imaging of disease sites, such as tumors, in a patient.

The diagnostic antibody may be associated with one or more detectable labels to facilitate detection and/or quantification of the bound antigen. Such labels may include, but are not limited to, fluorescent substances, biotin moieties and/or enzymes. For example, enzymes may include peroxidase such as horseradish peroxidase (HRP), alkaline phosphatase, etc., fluorescent substances may include FITC, RITC, etc. In addition, radioactive isotope labels, latex bead labels, colloid labels, biotin labels, etc. may be used, but are not limited thereto.

## Polypeptide conjugate

As described above, the moiety (second moiety) conjugated to a polypeptide, such as a therapeutic polypeptide, antibody or a fragment thereof, may include, but is not limited to, a drug, a detectable label, a small molecule, a polymer, etc. It may also be referred to as, but is not limited to, "payload" or "cargo."

Drugs include, but are not limited to, polypeptides, compounds, small molecules, extracts, nucleic acids, etc.

Small molecules refer to, but are not limited to, compounds that exhibit pharmaceutical activity and generally have a molecular weight of about 800 Da or less or 2000 Da or less. Inorganic small molecules refer to molecules that do not contain carbon atoms, and organic small molecules refer to compounds that contain at least one carbon atom.

Specific examples of detectable labels include, but are not limited to, fluorescent molecules (e.g., autofluorescent molecules, molecules that fluoresce upon contact with a reagent, etc.), radioactive labels (e.g., $^{111}$In, $^{125}$I, $^{131}$I, $^{212}$B, $^{90}$Y, $^{186}$Rh, etc.), biotin (e.g., detected through the reaction of biotin and avidin); fluorescent tag; contrast reagent, etc. Detectable labels also include peptides or polypeptides that can be detected by binding of detectably labeled antibodies or detection of bound antibodies via a sandwich-type assay. Additional examples of detectable labels include, but are not limited to, dye labels (e.g., chromophores, fluorophores, such as, but not limited to, Alexa Fluor™ fluorescent dyes (e.g., Alexa Fluor™ 350, 405, 430, 488, 532, 546, 555, 568, 594, 595, 610, 633, 635, 647, 660, 680, 700, 750, 790, etc.), Biophysical probes (spin labels, nuclear magnetic resonance (NMR) probes), Förster resonance energy transfer (FRET) type labels (e.g., at least one member of a FRET pair including at least one member of a fluorophore/quencher pair), bioluminescence resonance energy transfer (BRET)-type labels (e.g., at least one member of the BRET pair), immunodetectable tags (e.g., FLAG, His(6), etc.), localized tags, etc.

Polymers include, but are not limited to, water-soluble polymers. For example, polyethylene glycol (PEG) can be conjugated to a polypeptide to increase the serum half-life or decrease immunogenicity of the polypeptide. Additionally, it includes dextran and dextran derivatives, including dextran sulfate, P-amino cross linked dextrin, and carboxymethyl dextrin, cellulose and cellulose derivatives, including methylcellulose and carboxymethyl cellulose, starch and dextrines, and derivatives and hydroylactes of starch, polyalklyene glycol and derivatives thereof, including polyethylene glycol, methoxypolyethylene glycol, polyethylene glycol homopolymers, polypropylene glycol homopolymers, copolymers of ethylene glycol with propylene glycol, wherein said homopolymers and copolymers are unsubstituted or substituted at one end with an alkyl group, heparin and fragments of heparin, polyvinyl alcohol and polyvinyl ethyl ethers, polyvinylpyrrolidone, aspartamide, and polyoxyethylated polyols, dextran and dextran derivatives, dextrine and dextrine derivatives.

For the purposes herein, the moiety (second moiety) conjugated to the polypeptide may be linked (e.g., covalently linked) to an aptamer containing oxanine, and thus the polypeptide and the second moiety can be conjugated indirectly through an aptamer containing oxanine.

As representative embodiments of polypeptide conjugates herein, antibody-drug conjugates (ADCs) and Fc fragment-drug conjugates are described below.

### *Antibody-drug conjugate (ADC)*

The term "antibody-drug conjugate, ADC" refers to an antibody to which a therapeutically active substance or an active pharmaceutical ingredient (API) has been covalently coupled, such that the therapeutically active substance or an active pharmaceutical ingredient (API) can be targeted to the binding target of the antibody to exhibit its pharmacologic function. The therapeutically active substance or an active pharmaceutical ingredient may be a cytotoxin that is able to kill malignant or cancer cells, or an antibiotic, an enzyme such as nuclease, or a radionuclide, but is not limited thereto. For the purposes herein, a therapeutically active substance or an active pharmaceutical ingredient is attached (e.g., covalently linked) to an aptamer containing oxanine, and oxanine forms a covalent bond with the lysine residue of the antibody at a specific site of the antibody that is specifically recognized by the aptamer, and as a result, the therapeutically active substance or active pharmaceutical ingredient can be conjugated to the desired specific site of the antibody.

The terms "cytotoxin" and "cytotoxic agent" refer to any molecule that inhibits or prevents the function of cells and/or causes destruction of cells (cell death), and/or exerts antiproliferative effects. It will be appreciated that a cytotoxin or cytotoxic agent of an ADC also is referred to in the art as the "payload" of the ADC. A number of classes of cytotoxic agents are known in the art to have potential utility in ADC molecules and can be used in the ADC described herein. Such classes of cytotoxic agents include, microtubule structure formation inhibitors, mitosis inhibitors, topoisomerase inhibitors, or DNA intercalators, but are not limited thereto. Examples of specific cytotoxic agents include maytansinoid, auristatin, dolastatin, trichothecene, CC-1065 drug (NSC 298223), calicheamicin, enediynes, taxane, anthracycline, methotrexate, adriamycin, vindesine, vinca alkaloid, doxorubicin, melphalan, mitomycin C, chlorambucil, daunorubicin, daunomycin, etoposide, teniposide, carminomycin, aminopterin, dactinomycin, bleomycin, esperamicin, 5-fluorouracil, nitrogen mustar (mechlorethamine HCL), cis-platinum and its analogs, cisplatin, CPT-11, or docetaxel, but are not limited thereto.

*Fc fragment-Drug Conjugate*

"Drug", which forms a conjugate with an Fc fragment, means a substance that exhibits therapeutic activity when administered to humans or animals, and includes, but is not limited to, polypeptides, compounds, small molecules, extracts, nucleic acids, and the like. Polypeptide drugs may include hormones (e.g., intestinal hormones, growth hormone-releasing hormones, growth hormone-releasing peptides, etc.), interferons (e.g., interferon-$\alpha$, -$\beta$, -$\gamma$, etc.), interleukins, growth factors, granulocyte colony stimulating factor (G-CSF), granulocyte-macrophage colony stimulating factor (GM-CSF), glucacon-like peptides (e.g., GLP-1, etc.), erythropoietin, enzymes, etc., but are not limited thereto. In addition, any derivative may be also included in the scope of the polypeptide drug of the present invention, as long as it has a function, structure, activity or stability substantially equal to or increased to that of the natural form of the polypeptide drug.

In addition to the polypeptide drugs, other drugs may also be linked to the Fc fragment. Non-limiting examples of these drugs include antibiotics selected from among derivatives and mixtures of tetracycline, minocycline, doxycycline, ofloxacin, levofloxacin, ciprofloxacin, clarithromycin, erythromycin, cefaclor, cefotaxime, imipenem, penicillin, gentamycin, streptomycin, vancomycin, and the like; anticancer agents selected from among derivatives and mixtures of methotrexate, carboplatin, taxol, cisplatin, 5-fluorouracil, doxorubicin, etoposide, paclitaxel, camtotecin, cytosine arabinoside, and the like; anti-inflammatory agents selected from among derivatives and mixtures of indomethacin, ibuprofen, ketoprofen, piroxicam, probiprofen, diclofenac, and the like; antiviral agents selected from among derivatives and mixtures of acyclovir and robavin; and antibacterial agents selected from among derivatives and mixtures of ketoconazole, itraconazole, fluconazole, amphotericin B and griseofulvin.

In general, the Fc region is in the form of a dimer of a heavy chain fragment composed of heavy chain $C_{H2}$ and $C_{H3}$ domains, and may include a hinge portion in some cases. The Fc fragment herein may contain a portion or the all the heavy-chain constant region 1 ($C_{H1}$) and/or the light-chain constant region 1 ($C_{L1}$), except for the variable regions of the heavy and light chains. Further, it may be a region from which part of the amino acid sequence corresponding to $C_{H2}$ and/or $C_{H3}$ is removed. The Fc fragment herein may include a native amino acid sequence and sequence derivatives (mutants) thereof. An amino acid sequence derivative is a sequence that is different from the native amino acid sequence due to a deletion, an insertion, a non-conservative or conservative substitution or combinations thereof of one or more amino acid residues. In addition, the Fc fragment herein may be in the form of having native sugar chains, increased sugar chains compared to a native form or decreased sugar chains compared to the native form, or may be in a deglycosylated form.

The Fc fragment herein may be homodimeric or heterodimeric. In general, homodimerization of the Fc fragment is induced by a non-covalent bond between the last domains of an antibody constant region ($C_{H3}$ domain in for IgG) and a disulfide bond between hinge regions. Heterodimeric Fc fragment can be generated through engineering so as to have a bond where heterodimerization is preferred and homodimerization is not preferred or inhibited, via a specific non-covalent bond between the last domains of a constant region that greatly contribute to homodimerization of naturally occurring antibodies. For example, through gene manipulation, mutations are induced in CH3 domains of two different antibody heavy chains so that the two heavy chains are very similar in structure to naturally occurring antibodies, have a minimal deviation in sequence, and form a heterodimer. Examples of technologies related to this include a knob-into-hole technology available from Genentech, ZW1 from Zymeworks, HA-TF available from XENECORE Co., Ltd, SEEDbody available from EMD Serono, and the like, but are not limited thereto.

## Formulation, Composition and Method

The conjugate of the present disclosure can be formulated in a variety of different ways. In general, where the conjugate is a polypeptide-drug conjugate, the conjugate is formulated in a manner compatible with the drug conjugated to the polypeptide, the condition to be treated, and the route of administration to be used. The conjugate (e.g., polypeptide-drug conjugate) can be provided in any suitable form, e.g., in the form of a pharmaceutically acceptable salt, and can be formulated for any suitable route of administration, e.g., oral, topical or parenteral administration. Where the conjugate is provided as a liquid injectable (such as in those embodiments where they are administered intravenously or directly into a tissue), the conjugate can be provided as a ready-to-use dosage form, or as a reconstitutable storage-stable powder or liquid composed of pharmaceutically acceptable carriers and excipients.

Methods for formulating conjugates can be adapted from those available in the art. For example, conjugates can be provided in a pharmaceutical composition comprising a therapeutically effective amount of a conjugate and a pharmaceutically acceptable carrier (e.g., saline). The pharmaceutical composition may optionally include other additives (e.g., buffers, stabilizers, preservatives, and the like). In some embodiments, the formulations are suitable for administration to a mammal, such as those that are suitable for administration to a human.

When the conjugate herein is used for therapeutic purposes, administration of a composition comprising the conjugate may prevent a disease, or inhibit, stop or delay the onset or progression of a disease state, or ameliorate or beneficially alter symptoms.

Accordingly, the present disclosure also provides a therapeutic composition comprising an effective amount of the conjugate herein and a treatment method comprising administering the same to a subject.

The term "effective amount" refers to an amount sufficient to achieve the desired result, e.g., an amount effective to treat or prevent a disease, when administered to subjects, including humans. The effective amount may vary depending on various factors such as a formulation method, administration mode, a patient's age, body weight, sex, disease severity, diet, administration time, administration route, excretion rate, and response sensitivity. Administration dosage or therapeutic regimen may be adjusted to provide an optimal therapeutic response as will be understood by those skilled in the art.

The composition of the present disclosure may be provided together with one or more additives selected from the group consisting of pharmaceutically acceptable carriers, diluents, and excipients.

The pharmaceutically acceptable carrier, which is commonly used in the formulation of antibody, may be one or more selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, etc., but is not limited thereto. The composition may further include one or more selected from the group consisting of diluents, excipients, lubricants, wetting agents, sweeteners, flavoring agents, emulsifiers, suspending agents, preservatives, etc., which are commonly used in the preparation of pharmaceutical compositions, in addition to the above components. The pharmaceutically acceptable carriers and formulations suitable for the present disclosure, including those exemplified above, are described in detail in the literature [Remington's Pharmaceutical Sciences, latest edition].

The composition may be administered orally or parenterally. When administered parenterally, intravenous injection, subcutaneous injection, intramuscular injection, intraperitoneal injection, endothelial administration, topical administration, intranasal administration, intraocular administration, intrathecal administration, intrathecal administration, intracranial administration, and intrastriatal administration may be used.

In some embodiments, the composition is provided as a sterile liquid preparation, for example, as an isotonic aqueous solution, a suspension, an emulsion, a dispersion, or a viscous composition, which, in some aspects, may be buffered to a selected pH. Liquid preparations are normally easier to prepare than gels, other viscous compositions, and solid compositions. In addition, liquid compositions are particularly convenient to administer by injection. Viscous compositions, on the other hand, may be formulated within the appropriate viscosity range to provide longer contact periods with a specific tissue. Liquid or viscous compositions may include a carrier which may be a solvent or dispersion medium containing, for example, water, saline, phosphate buffered saline, polyol (e.g., glycerol, propylene glycol, liquid polyethylene glycol) and appropriate mixtures thereof.

Sterile injectable solutions may be prepared by incorporating the binding molecule in a solvent, such as in admixture with a suitable carrier, diluent, or excipient such as sterile water, physiological saline, glucose, dextrose, or the like. The compositions may also be lyophilized. The compositions may include auxiliary substances such as wetting, dispersing, or emulsifying agents (e.g., methylcellulose), pH buffering agents, gelling or viscosity enhancing additives, preservatives, flavoring agents, colors, etc., depending upon the route of administration and the desired preparation.

Various additives which enhance stability and sterility of the compositions, including antimicrobial preservatives, antioxidants, chelating agents, and buffers, may be added. Prevention of microbial actions may be ensured by various antibacterial and antifungal agents, for example, parabens, chlorobutanol, phenol, sorbic acid, etc. Prolonged absorption of the injectable pharmaceutical form may be brought about by the use of agents delaying absorption, for example, aluminum monostearate and gelatin.

## EXAMPLES

Hereinafter, the present invention will be described in more detail with reference to the following examples. However, these examples are only for illustrating the present invention, and the scope of the present invention is not limited by these examples.

### Example 1. Site-specific bioconjugation to thrombin

#### 1-1. Preparation of aptamers containing oxanine

The present inventors have established the enzymatic strategy to incorporate oxanine inside aptamers using 2'-deoxyoxanosine triphosphates (dOTPs) and polymerases (Therminator™ DNA polymerase). Specifically, to prepare dOTP, 10 mg of dGTP was added to 2.6 mL of distilled water, then 1 mL of 4 M acetate buffer (pH 3.7, final 1 M) was added. After incubation for 5 min at 40 °C, the mixture was mixed with 0.4 mL of 1 M $NaNO_2$ (final 100 mM). After incubation for 4 h at 40 °C, the mixture was neutralized by adding NaOH to stop the reaction. dOTP was purified by HPLC purification (Shimadzu, Japan, Column: Ascentis® Express 90 Å AQ-C18, 2.7 μm HPLC Column 100 × 4.6 mm; gradient buffer system: 0% ACN in 100 mM TEAA at 0 min to 20% ACN in 100 mM TEAA at 20 min, 1 mL/min flow rate) and lyophilized.

dOTPs were incorporated into the aptamer strand using Therminator™ DNA polymerase and the template DNA containing cytosine. As oxanine is a guanosine analogue that forms hydrogen bonds with cytosine, dOTP can be incorporated into the aptamer strand via DNA chain extension using a cytosine-containing DNA template, DNA polymerase, and primers from the 5' end to the desired position. The present inventors confirmed that dOTPs can be inserted into DNA, RNA, or even modified DNA/RNA hybrid strand, indicating the availability of various aptamers for this approach. Moreover, the position and number of dOTPs to be inserted can be flexibly designated by designing template sequence. dNTPs can further be included if required without any purification in between. After polymerase reaction, template DNA was removed by template replacement and HPLC to produce only the single-stranded aptamer containing oxanine.

In particular, for synthesis of reactive aptamers with oxanine at the 3'-end, all components, including the aptamer (final 2 $\mu$M), template DNA (final 2.4 $\mu$M), dOTP (final 100 $\mu$M), 100mM MgCl$_2$ (final 8 mM), 10 $\times$ ThermoPol® reaction buffer (final 1$\times$, NEB-provided), and Therminator™ DNA polymerase (24 units; as defined by NEB Co.), were mixed in distilled water to a final volume of 500 $\mu$L. The 1$\times$ ThermoPol buffer was composed of 20 mM Tris-HCl (pH 8.8), 10 mM (NH$_4$)$_2$SO$_4$, 10 mM KCl, 2 mM MgSO$_4$, and 0.1% Triton® X-100. After incubation at 37 °C for 2 hours, cDNA (final 2.88 $\mu$M) that is fully complementary to the template DNA was added, and the mixture was heated at 95 °C for 5 min to generate single strand aptamer with oxanine. All the other components, beside the oxanine-including aptamers, were then removed by HPLC purification.

**1-2. Site-specific bioconjugation to thrombin**

Human-alpha thrombin and its well-known aptamers, HD1 (15-mer length of aptamer that binds to exosite I of thrombin; 5'-GGTTGGTGTGGTTGG-3'; SEQ ID NO: 1) and HD2 (29-mer length of aptamer that binds to exosite II of thrombin; 5'-AGTCCGTGGTAGGGCAGGTTGGGGTGACT-3'; SEQ ID NO: 2) were used. Each aptamer labeled with fluorescent dye at 5'-terminus and 10 dOTPs at 3'-terminus was prepared (See Example 1-1) and mixed with thrombin. Specifically, thrombin (1.25 $\mu$M), NaCl (100mM), MgCl$_2$ (5mM), KCl (5mM), Tween-20 (0.01% v/v), and oxanine-inserted HD1 (1 equiv. relative to thrombin) and HD2 (2 equiv. relative to thrombin) in HEPES (50mM pH 8.4) were mixed at 37°C for 1 day.

Whether the aptamer brings oxanine close to the amine group of the target protein, enabling proximity-enhanced covalent bond formation, was monitored with SDS-PAGE (protein bands) and electrophoresis (DNA bands). As a comparison group, aptamers HD1 and HD2 without oxanine introduced, or random DNA (not aptamer) with oxanine introduced, were used, and an untreated control group without any treatment with thrombin was also used for comparison.

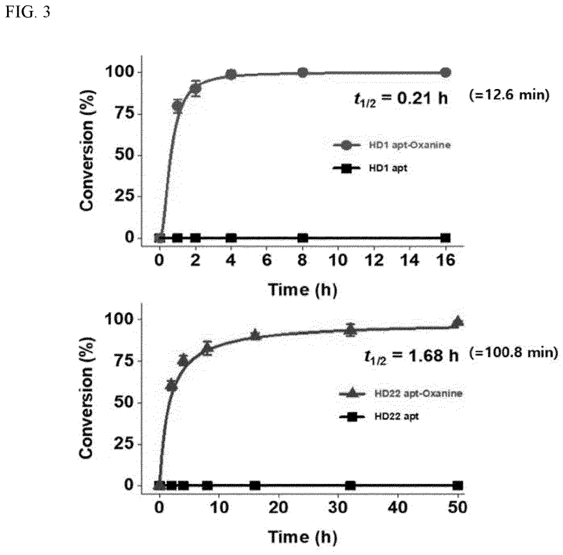

As a result, as shown in Fig. 2, in both cases where an aptamer without oxanine was used (lane 2, 4) and where random DNA (not aptamer) with oxanine introduced was used (lane 6), none of the covalent bond formation between thrombin and DNA was observed, similar to the untreated control (lane 1). However, in the presence of both oxanine-containing aptamers HD1 and HD22 (lanes 3 and 5), >95% 1 to 1 conjugation was achieved. Further, as shown in Fig. 3, HD1 reached 50 % modification rate (i.e., rate of conversion of aptamer to aptamer-thrombin conjugate) within just 12.6 minute while HD22 reached 50 % modification rate within 1.68 hours. Therefore, it was confirmed that by using an aptamer containing oxanine, covalent bonding was induced in the native protein in a short period of time and with very high efficiency.

**Example 2. Site-specific bioconjugation to PTK7**

A membrane receptor protein, PTK7 and its well-known aptamer, Sgc8c (5'-ATCTAACTGCTGCGCCGCCGGGAAAATACTGTACGGTTAGA-3'; SEQ ID NO: 3) were used. The aptamer labeled with fluorescent dye at 5'-terminus and 10 dOTPs at 3'-terminus was prepared (See Example 1-1) and mixed with PTK7. Specifically, PTK7 (1.25$\mu$M), NaCl (100mM), MgCl$_2$ (5mM), KCl (5mM), Tween-20 (0.01% v/v), and oxanine-containing Sgc8c (5 equiv. relative to PTK7) in HEPES (50mM pH 8.4) were mixed at 37°C for 1 day. Whether the aptamer brings oxanine close to the amine group of the target protein, enabling proximity-enhanced covalent bond formation, was monitored with SDS-PAGE (protein bands) and electrophoresis (DNA bands). Comparison was made with the untreated control group in which no treatment was performed on PTK7.

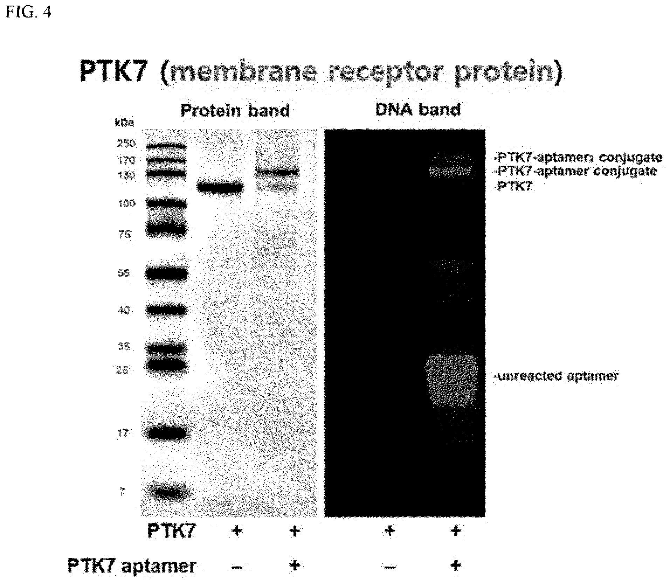

As a result, as shown in Fig. 4, the oxanine-containing aptamer Sgc8c formed PTK7-aptamer conjugation with a yield of 85.4%.

**Example 3. Site-specific bioconjugation to nucleolin**

A tumor-related nucleolar protein, nucleolin and its well-known aptamer, AS1411 (5'-GGTGGTGGTGGTTGTGGTGGTGGTGG-3'; SEQ ID NO: 4) were used. The aptamer labeled with fluorescent dye at 5'-terminus and 10 dOTPs at 3'-terminus was prepared (See Example 1-1) and mixed with nucleolin. Specifically, nucleolin (1.25$\mu$M), NaCl (100mM),

MgCl$_2$ (5mM), KCl (5mM), Tween-20 (0.01% v/v), and oxanine-containing AS1411 (4 equiv. relative to nucleolin) in HEPES (50mM pH 8.4) were mixed at 37°C for 1 day. Whether the aptamer brings oxanine close to the amine group of the target protein, enabling proximity-enhanced covalent bond formation, was monitored with SDS-PAGE (protein bands) and electrophoresis (DNA bands). Comparison was made with the untreated control group in which no treatment was performed on nucleolin.

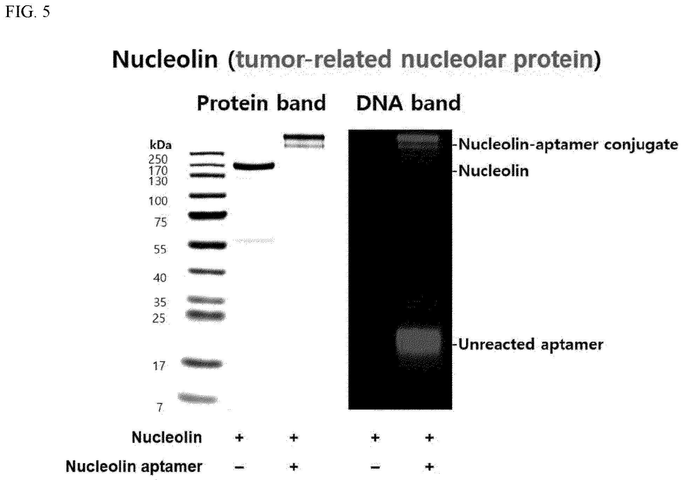

As a result, as shown in Fig. 5, the oxanine-containing aptamer AS1411 formed nucleolin-aptamer conjugation with a yield of >99%.

**Example 4. Site-specific bioconjugation to Fc dimer protein**

Fc dimer protein and its well-known aptamer, Fc RNA aptamer Apt8-2 (5'-ggAGG[2'-F-rU]Gc[2'-F-rU]ccgaaaGgaa[2'-F-rC][2'-F-rU]cc-3'; Lowercase letters are DNA, uppercase letters are RNA; SEQ ID NO: 5) (Y Nomura et al., Nucleic Acids Research, Volume 38, Issue 21, 1 November 2010, Pages 7822-7829) were used. The aptamer labeled with fluorescent dye at 5'-terminus and 10 dOTPs at 3'-terminus was prepared (See Example 1-1) and mixed with the Fc protein. Specifically, the Fc protein (1.25μM), NaCl (100mM), MgCl$_2$ (5mM), KCl (5mM), Tween-20 (0.01% v/v), and oxanine-containing Fc RNA aptamer (4 equiv. relative to the Fc protein) in HEPES (50mM pH 8.4) were mixed at 37°C for 1 day. Whether the aptamer brings oxanine close to the amine group of the target protein, enabling proximity-enhanced covalent bond formation, was monitored with SDS-PAGE (protein bands) and electrophoresis (DNA bands). Comparison was made with the untreated control group in which no treatment was performed on the Fc protein.

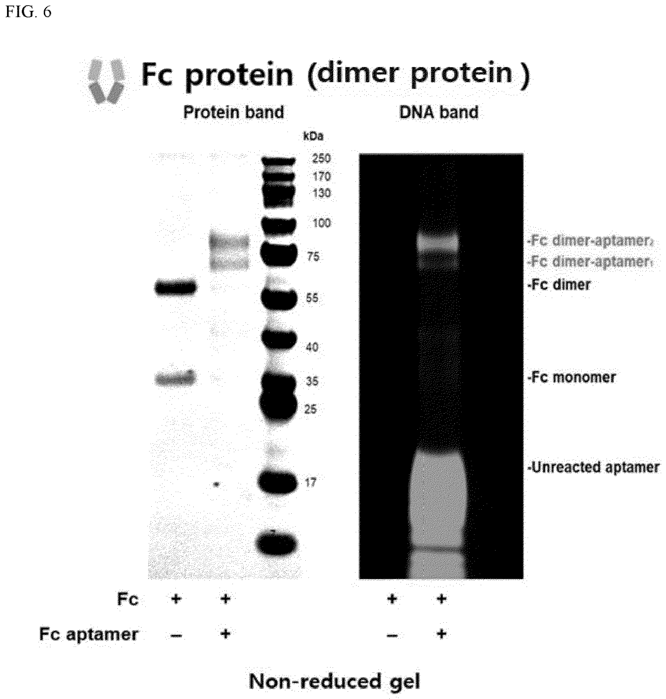

As a result, as shown in Fig. 6, it is observed that one or two aptamers coupled to the Fc dimer protein, and it is likely to be due to the steric effects of Fc dimerization that hinders the coupling reaction between the Fc monomer and aptamer. However, at least one formed conjugation with the Fc dimer protein with a yield of >98%.

**Example 5. Site-specific bioconjugation for an antibody**

Trastuzumab, which is a monoclonal antibody used to treat breast cancer and stomach cancer, and Fc RNA aptamer Apt8-2 (same as Example 4) were used. The aptamer labeled with fluorescent dye at 5'-terminus and 10 dOTPs at 3'-terminus was prepared (See Example 1-1) and mixed with trastuzumab. Specifically, trastuzumab (1.25μM), NaCl (100mM), MgCl$_2$ (5mM), KCl (5mM), Tween-20 (0.01% v/v), and oxanine-containing Fc RNA aptamer (4 equiv. relative to trastuzumab) in HEPES (50mM pH 8.4) were mixed at 37°C for 1 day. Whether the aptamer brings oxanine close to the amine group of the target protein, enabling proximity-enhanced covalent bond formation, was monitored with SDS-PAGE (protein bands) and electrophoresis (DNA bands). Comparison was made with the untreated control group in which no treatment was performed on trastuzumab.

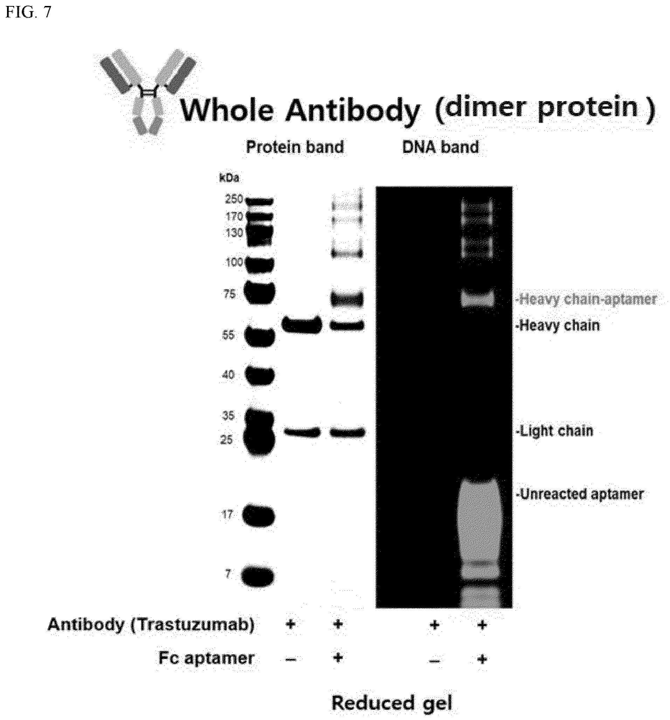

As a result, as shown in Fig. 7, antibody-aptamer conjugate was detected only at the heavy chain, where Fc domain locates, and no conjugation was formed at the light chain, indicating its ultra-high specificity. As trastuzumab is a also homodimer, no complete band shift of heavy chain was observed, however, at least one modification per antibody was detected in consensus with the Fc protein coupling.

**Example 6. Preparation of site-specific antibody-drug conjugate using aptamer containing oxanine and evaluation of its activity**

**6-1. Preparation of antibody-drug conjugate**

Trastuzumab, which is a monoclonal antibody used to treat breast cancer and stomach cancer, and Fc RNA aptamer Apt8-2 (same as Example 4) were used. The antibody-aptamer conjugate without drug conjugation were prepared as described in Example 5. The process of conjugating a drug to the antibody-aptamer conjugate is as follows: DM1, a cytotoxic agent that inhibits microtubulin, was used as the drug. The DBCO-modified DM1 drug was attached to DNA that was prepared by modifying the DNA sequence complementary to the aptamer Apt8-2 with one azide (i.e., 5'-GGA GTT CCT TTC GGA GCA CCT CC [azide]-3'; SEQ ID NO: 6), using the Strain-promoted azide-alkyne cycloaddition (SPAAC) click reaction. Since the DNA is a complementary sequence to the Fc RNA aptamer Apt8-2, when these two oligonucleotides are together, they hybridize into a duplex through self-assembly. Thus, DM1 can be site-specifically conjugated to trastuzumab by mixing DNA-DM1 and Trastuzumab-Apt8-2 conjugate in 1X PBS and gently agitating for about 10 minutes.

Whether the drug was successfully conjugated to the antibody-aptamer conjugate was confirmed by SDS-PAGE (protein band) and electrophoresis (DNA band). As a result, as shown in Fig. 8, it was confirmed that a 1:1 antibody-drug conjugate was produced with a binding efficiency of ~100%. As Cy3 dye (green) was labeled at the 5' end of aptamer Apt8-2 and oxanine was introduced at the 3' end, "Fc apt-Oxa" in Fig. 8 shows that the aptamer Apt8-2 is attached to trastuzumab. Further, as Cy5 dye (red) was labeled at the 5' end of the DNA sequence (SEQ ID NO: 6) that is complementary to the aptamer Apt8-2 and oxanine was introduced at the 3' end, "DNA-DM1" shows that not only Apt8-2 but also the complementary DNA sequence and DM1 are attached to trastuzumab. "Merge" shows a yellow color in which both Cy3 and Cy5 are visible, as both Apt8-2 and DM1-DNA signals appear at the location of the trastuzumab protein band. It indicated that a 1:1 antibody-drug conjugate was produced with a binding efficiency of ~100%.

## 6-2. Comparison of antibody activities of antibody-drug conjugate

As a site-specific antibody-drug conjugate was prepared using an aptamer containing oxanine, it was evaluated whether the representative main functions of the antibody were maintained. The representative main functions that antibodies originally have are (i) Antigen binding ability, which is a function of an antibody recognizing an antigen, (ii) FcγR binding ability (FcγRIIIa binding ability), an important function for an antibody to cause an immune response, (iii) FcRn binding ability, an important function for an antibody to exist in the body for a long time.

The ELISA method to confirm this is as follows: We coated a 96-well polystyrene ELISA plate (Costar) with 50 $\mu$l of 4 $\mu$g/ml antigen (HER2/FcγRIIIa/FcRn) resuspended in 0.05 M Na$_2$CO$_3$ (pH 9.6) in for coating and then incubated the plate overnight at 4 °C. After blocking each well of the plate with 100 $\mu$l of 1 × PBS and 4% skim milk, the plate was incubated at room temperature for 1 h, washed four times with 1 × PBS containing 0.05% Tween20 (0.05% PBST), and then 50 $\mu$l of serially diluted, purified bispecific antibodies was added. The plate was then incubated for 1 h at room temperature, washed four times with 0.05% PBST, and treated with 50 $\mu$l of 1:5000 diluted goat anti-human IgG (H + L)-HRP conjugate. After another hour of incubation at room temperature and four washes in 0.05% PBST, ELISA signals were developed by adding 50 $\mu$l of 1-Step™ Ultra-TMB solution and incubating the plate at room temperature. Next, 50 $\mu$l of 2 M H$_2$SO$_4$ was added to each well to quench the signals, and the absorbance was measured at 450 nm using a plate reader (BioTek).

Fig. 9, which shows the results of analysis of binding activity to the antigen, HER2, through ELISA, indicates that there was no significant difference in the HER2 binding activity of "trastuzumab-DNA", in which an aptamer was conjugated to trastuzumab, and "trastuzumab-DNA-DM1", in which DM1 is conjugated to trastuzumab through an aptamer, compared to "Trastuzumab". This means that the antigen binding domain of the antibody was not disturbed even though the drug was attached by site-specific conjugation of the Fc aptamer.

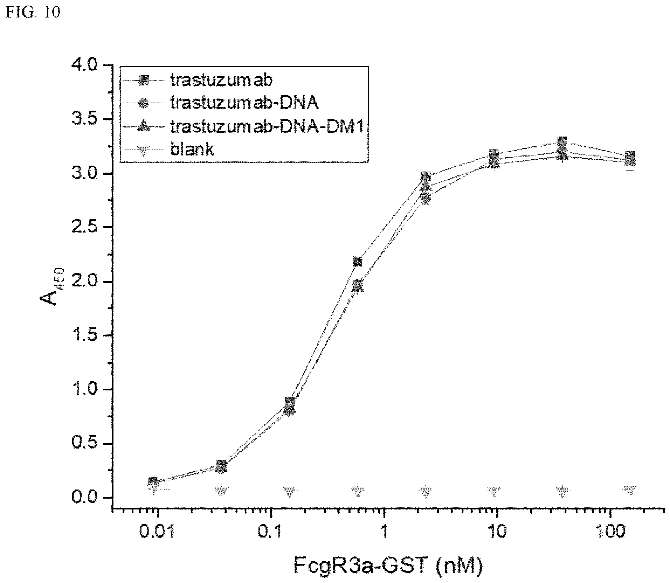

Fig. 10, which shows the results of analyzing the binding activity for FcγR, in particular FcγRIIIa, through ELISA, indicates that there was no significant difference in the binding activity of trastuzumab-DNA and trastuzumab-DNA-DM1 compared to trastuzumab. This means that the FcγR binding domain was not disturbed even though the drug was attached by site-specific conjugation of the Fc aptamer. FcγR binding plays a role in an immune response by causing antibody-dependent cell-mediated cytotoxicity (ADCC).

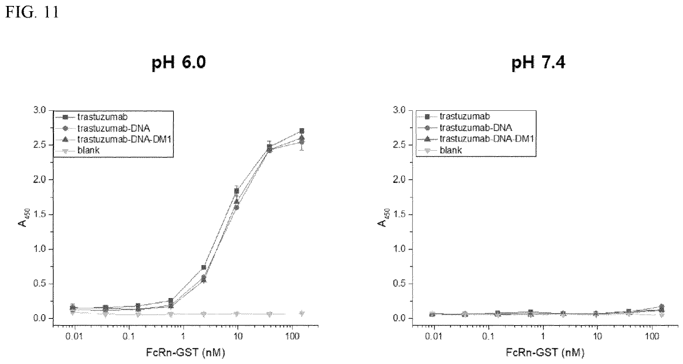

Fig. 11, which shows the results of analyzing pH-dependent FcRn binding activity through ELISA, indicates that there is no significant difference in the binding activity of trastuzumab-DNA and trastuzumab-DNA-DM1 compared to trastuzumab. The characteristic of binding to FcRn at pH 6.0 and not binding at pH 7.4 plays an important role in allowing antibodies to penetrate cells. This is one of the reasons why antibodies can have a long half-life without being degraded in lysosomes.

In conclusion, it was confirmed that despite the site-specific conjugation of the aptamer or the drug-conjugated aptamer, the representative main functions of the antibody were maintained.

## 6-3. Analysis of target cell death effect of antibody-drug conjugate

"Rituximab" as an isotype control, "Trastuzumab" as a model antibody, "Trastuzumab-DNA" in which an aptamer is conjugated to trastzumab, and "Trastuzumab-DNA-DM1" in which DM1 is conjugated to trastzumab through an aptamer were administered at different concentrations (20 nM, 10 nM, 5 nM) to SKBR-3 cells, which are HER2 overexpressing cells, and cell death was observed.

Real-time cytotoxic effects of the antibody constructs on cancer cell lines were studied using the xCELLigence® RTCA instrument (ACEA Biosc Inc.) at 37°C and 5% CO$_2$. In particular, 50 $\mu$L of cell culture media was added to all wells on the E-plate 16 plates and a short baseline measurement was recorded by the instrument using the RTCA Software 1.0. Target cell lines (SKBR-3) were then seeded on the E-plates. 50 $\mu$L of antibody diluted in cell culture media and 50 $\mu$L of freshly isolated effector PBMCs were added to appropriate wells and measurements were recorded every 10 min for 96 h. Cell index (CI) values obtained were normalized to the CI values recorded after 24 h of incubation. Data was plotted and analyzed using GraphPad Prism v. 8. Cytotoxicity was calculated following the equation:

$$\text{Cytotoxicity (\%)} = ((\text{Experiment value} - \text{Low control}) / (\text{Low control} - 1)) \times 100$$

(where low control consisted of target cells incubated with effector PBMC only without antibody constructs.)

As shown in Figs. 12 to 14, Trastuzumab and Trastuzumab-DNA showed similar decreases in cytolysis over time. In contrast, Trastuzumab-DNA-DM1 was observed to have target cell cytolysis of more than 90% due to the effect of DM1. Further, Trastuzumab-DNA-DM1 was observed to have high cytolysis even at low concentrations. In other words, when only the aptamer was conjugated to trastuzumab, there was no cytotoxic effect, but when DM1 was conjugated to trastuzumab through the aptamer, cytotoxicity was confirmed.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are intended to be embraced by the claims.

## Claims

1. A composition for preparing a conjugate of a polypeptide, the composition comprising an aptamer containing oxanine, wherein the aptamer specifically recognizes the polypeptide.

2. The composition of claim 1, wherein an amide bond is formed between the lysine residue present in the region recognized by the aptamer in the polypeptide and the oxanine contained in the aptamer.

3. The composition of claim 1, wherein the polypeptide is a therapeutic polypeptide, or an antibody or a fragment thereof.

4. The composition of claim 3, wherein the polypeptide is an enzyme, a cytokine, a chemokine, a growth factor, a hormone, a receptor, a tumor-related protein, a monoclonal antibody, a polyclonal antibody, a multispecific antibody, a synthetic antibody, a chimeric antibody, a humanized antibody, a human antibody, an antibody fusion protein, or a fragment of an antibody.

5. The composition of claim 1, wherein the aptamer is bound to a drug, a detectable label, a small molecule, or a polymer.

6. A method of preparing a conjugate of a polypeptide, comprising mixing the composition of any of claims 1 to 5 with a polypeptide.

7. A conjugate comprising a polypeptide and an aptamer containing oxanine, wherein the aptamer specifically recognizes the polypeptide.

8. The conjugate of claim 7, wherein an amide bond is formed between the lysine residue present in the region recognized by the aptamer in the polypeptide and the oxanine contained in the aptamer.

9. The conjugate of claim 7, wherein the polypeptide is a therapeutic polypeptide, or an antibody or a fragment thereof.

10. The conjugate of claim 9, wherein the polypeptide is an enzyme, a cytokine, a chemokine, a growth factor, a hormone, a receptor, a tumor-related protein, a monoclonal antibody, a polyclonal antibody, a multispecific antibody, a synthetic antibody, a chimeric antibody, a humanized antibody, a human antibody, an antibody fusion protein, or a fragment of an antibody.

11. The conjugate of claim 7, wherein the aptamer is bound to a drug, a detectable label, a small molecule, or a polymer.

12. A conjugate comprising a polypeptide and a drug, wherein the drug is conjugated to the polypeptide via an aptamer containing oxanine, and the aptamer specifically recognizes the polypeptide.

13. The conjugate of claim 12, wherein an amide bond is formed between the lysine residue present in the region recognized by the aptamer in the polypeptide and the oxanine contained in the aptamer.

14. The conjugate of claim 12, wherein the polypeptide is a therapeutic polypeptide, or an antibody or a fragment thereof.

15. The conjugate of claim 14, wherein the polypeptide is an enzyme, a cytokine, a chemokine, a growth factor, a hormone, a receptor, a tumor-related protein, a monoclonal antibody, a polyclonal antibody, a multispecific antibody, a synthetic antibody, a chimeric antibody, a humanized antibody, a human antibody, an antibody fusion protein, or a fragment of an antibody.

FIG. 1

FIG. 2

**Thrombin (enzyme)**

| | | | | | | | | | | | | |
|---|---|---|---|---|---|---|---|---|---|---|---|---|
| Thrombin | + | + | + | + | + | + | + | + | + | + | + | + |
| Exosite I aptamer | – | + | + | – | – | – | – | + | + | – | – | – |
| Exosite II aptamer | – | – | – | + | + | – | – | – | – | + | + | – |
| Random DNA | – | – | – | – | – | + | – | – | – | – | – | + |
| Oxanine | – | – | + | – | + | + | – | – | + | – | + | + |

thrombin-aptamer conjugates

-thrombin

unreacted aptamer

FIG. 3

FIG. 4

PTK7 (membrane receptor protein)

FIG. 5

**Nucleolin** (tumor-related nucleolar protein)

FIG. 6

FIG. 7

**Whole Antibody (dimer protein )**

Protein band    DNA band

-Heavy chain-aptamer
-Heavy chain
-Light chain
-Unreacted aptamer

Antibody (Trastuzumab)    +    +        +    +
Fc aptamer               −    +        −    +

**Reduced gel**

FIG. 8

FIG. 9

FIG. 10

FIG. 11

pH 6.0 / pH 7.4

FIG. 12

FIG. 13

FIG. 14

# EUROPEAN SEARCH REPORT

Application Number

EP 24 16 8704

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | OHTSUKA KEIICHI ET AL: "Fluorescence imaging of potassium ions in living cells using a fluorescent probe based on a thrombin binding aptamer-peptide conjugate", CHEMICAL COMMUNICATIONS, vol. 48, no. 39, 1 January 2012 (2012-01-01), page 4740, XP093192959, UK ISSN: 1359-7345, DOI: 10.1039/c2cc30536d * supplementary material; figure 1A * | 1-5 | INV. A61K47/54 C07H21/04 C12N15/115 A61K47/68 |
| Y | JANG EUI KYOUNG ET AL: "Nucleobase-involved native chemical ligation: a novel reaction between an oxanine nucleobase and N-terminal cysteine for oligonucleotide-peptide conjugation", CHEMICAL COMMUNICATIONS, vol. 56, no. 41, 1 January 2020 (2020-01-01), pages 5508-5511, XP093192925, UK ISSN: 1359-7345, DOI: 10.1039/C9CC08808C * the whole document * | 1-7, 9-12,14, 15 | |
| Y | JANG ET AL: "A versatile enzymatic labeling of reactive oxanine nucleobase and its applications as a latent crosslinker for DNA-DNA/DNA-peptide ligation", THESIS, GRADUATE SCHOOL, KOREA UNIVERSITY, 1 January 2018 (2018-01-01), pages 1-122, XP009526592, * the whole document * | 1-7, 9-12,14, 15 | |

**TECHNICAL FIELDS SEARCHED (IPC)**

A61K
C12N
C07H

-/--

The present search report has been drawn up for all claims

| Place of search | Date of completion of the search | Examiner |
|---|---|---|
| The Hague | 6 August 2024 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 24 16 8704

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JAN L. VINKENBORG ET AL: "Aptamer-Based Affinity Labeling of Proteins", ANGEWANDTE CHEMIE INTERNATIONAL EDITION, vol. 51, no. 36, 3 September 2012 (2012-09-03), pages 9176-9180, XP055047928, ISSN: 1433-7851, DOI: 10.1002/anie.201204174 * the whole document * | 6,7, 9-12,14, 15 | |

-----

| | | | TECHNICAL FIELDS SEARCHED (IPC) |
|---|---|---|---|

The present search report has been drawn up for all claims

| Place of search | Date of completion of the search | Examiner |
|---|---|---|
| The Hague | 6 August 2024 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230046232
- US 5660985 A
- US 5756703 A
- US 5270163 A
- US 5475096 A
- US 5705337 A
- US 5580737 A
- US 6376190 B
- US 7947447 B
- US 7855054 B
- US 8409795 B
- US 20070166740 A
- US 20090098549 A

**Non-patent literature cited in the description**

- **SPROAT et al.** *Nucle. Acid. Res.,* 1991, vol. 19, 733-738
- **COTTON et al.** *Nucl. Acid. Res.,* 1991, vol. 19, 2629-2635
- **HOBBS et al.** *Biochemistry,* 1973, vol. 12, 5138-5145
- *Angew Chem Int Ed Engl.,* 05 December 2016, vol. 55 (49), 15258-15262
- **Y NOMURA et al.** *Nucleic Acids Research,* 01 November 2010, vol. 38 (21), 7822-7829